# EUROPEAN PATENT APPLICATION

(11) **EP 1 724 357 A1**
(43) Date of publication of application: **22.11.2006**
(21) Application number: 05719780.8
(22) Date of filing: 02.03.2005
(51) Int. Cl.: C12P 23/00, A23D 9/007, A23L 1/30, C12R 1/89

(54) **A PROCESS FOR PRODUCING ASTAXANTHIN-CONTAINING LIPIDS**

(30) Priority: 04.03.2004 JP 2004060736
(71) Applicant: SUNTORY LIMITED, Osaka-shi, Osaka 530-8203 (JP)
(72) Inventor: Higashiyama, Kenichi, Kobe-shi, Hyogo 6580073 (JP); KAKIZONO, Toshihide, Higashi-hiroshima-shi, Hiroshima 7390023 (JP)
(74) Representative: Vossius & Partner
(86) International application number: PCT/JP2005/003465
(87) International publication number: WO 2005/085465

(57) **Abstract**

The present invention aims to provide a process which, by improving the conventional method of culturing the green alga *Haematococcus pluvialis,* promotes vegetative cell growth and astaxanthin biosynthesis so markedly as to enable efficient production of astaxanthin-containing lipids from the culture.

In order to attain this object, the green alga *Haematococcus pluvialis* is cultured with an organic nitrogen source being used in a medium at an AN/TN ratio of 65% or less, preferably 43% or less, more preferably 35% or less, to obtain algal bodies in which astaxanthin-containing lipids have been accumulated. If necessary, the astaxanthin-containing lipids may be extracted from the algal bodies and optionally purified.

## Description

### TECHNICAL FIELD

This invention relates to a process for producing astaxanthin-containing lipids characterized by cultivating the green alga *Haematococcus pluvialis* and collecting astaxanthin- containing lipids from the culture.

### BACKGROUND ART

Astaxanthin is a kind of red carotenoid which finds an extremely wide distribution in the animal kingdom as in the eggs or shells of crustaceans, the meat of salmon, and the epidermis of alfonsin, and it is involved in the development of the meat or bodily color. Uses of astaxanthin include its addition as a red pigment to feeds for trout and red sea bream in aquaculture (Seibutsu Kogakukai-shi 71(4):233-237 (1993)). Astaxanthin also has a potent anti-oxidation action, so its use as a pharmaceutical active ingredient is under review (Japanese Patent No. 3163127). Astaxanthin has been commercialized as a material for health foods (Food Style 21, 5(12):25-35 (2001)).

The green alga *Haematococcus pluvialis* accumulates a large amount of astaxanthin in its cells, so astaxanthin production by its cultivation has been commercialized (NatuRose Technical Bulletin #78, Cyanotech Corporation (2000); Food Style 21, 5(12):25-35 (2001)). The life cycle of *Haematococcus pluvialis* is characterized by the occurrence of vegetative cells and cysts. Vegetative cells are oval migratory cells each having two flagella and a gelatin-like cell wall. The astaxanthin content in vegetative cells is as low as about 10 pg/cell (J. Ferment. Technol. 74(1):17-20 (1992)). Cysts (also known as aplanospores or akinetes) are spherical cells that are characterized by having a hard cell wall within gelatinous matter but not flagella. Vegetative cells can be clearly distinguished from cysts by external observation. Cysts that have just grown from vegetative cells are low in astaxanthin content, have a brown or reddish brown tone, and are called brown-red immature cysts. The astaxanthin content in brown-red immature cysts is about 30 pg/cell (Biotechnol. Lett. 19(6):507-509 (1997)). By properly adjusting the culture conditions, the astaxanthin content in cyst cells increases with culture time, causing a tonal change to red, and the resulting cysts are called red mature cysts. Certain cysts have been reported to have an astaxanthin content as high as 613 pg/cell (Biotechnol. Lett. 16(2):133-138 (1994)).

The conditions for astaxanthin production by cultivation of *Haematococcus pluvialis* have mostly been studied with cultivation in the light accompanied by irradiation with light, and industrial production of astaxanthin is being performed by a method that cultivates *Haematococcus pluvialis* in an outdoor pond under sunlight or by a method that cultivates *Haematococcus pluvialis* in an outdoor dome under sunlight (Food Style 21, 5(12):25-35 (2001)). Green algae generally depend on photosynthesis to obtain energy, so it has been believed that they are not capable of growing or producing astaxanthin if not under light conditions (Seibutsu Kogakukai-shi, 71(4):233-237 (1993)). Those cultivation techniques using sunlight have the advantage of being low in the energy cost for irradiation, as well as in the initial cost of the irradiator; on the other hand, provision must be made for preventing microbial contamination in the outdoor system. If culture is to be performed indoors with the aid of light, the high energy cost for irradiation and the high initial cost of the irradiator pose a problem. Various R&D efforts are being made to reduce those costs. For example, with a view to increasing the efficiency of irradiation with light, it has been reported to perform culture using a tube reactor (J. Appl. Phycol. 5:593-604 (1993)) or an air-lift photoreactor (J. Ferment. Bioeng. 82:113-118 (1996)). However, those techniques still need to be improved before they can be commercialized.

With a view to solving the aforementioned problems, culture in the dark which does not depend on light has been studied, revealing that even in the dark, the green alga could be grown as vegetative cells by using acetic acid as a carbon source and that astaxanthin was accumulated within the cells as they grew (J. Ferment. Bioeng. 74:17-20 (1992)). It was also found that even cultivation in the dark could form cysts by a method of increasing the salt concentrations in the medium (Biotechnol. Lett. 19(6):507-509 (1997)) or by adopting aeration or some other means to create aerobic conditions (see commonly assigned Japanese Patent Application 2002-294420).

In the cultivation of *Haematococcus pluvialis* which is performed to produce astaxanthin, inorganic nitrogen is primarily used as the required nitrogen source in the medium for cell construction and exemplary nitrogen sources that have been used in the medium include nitrates (Appln. Microbiol. Biotechnol. 53:530-535 (2000)) and urea (Appln. Microbiol. Biotechnol. 53:537-540 (2001)). On the other hand, organic nitrogen sources such as high-protein nitrogen sources including defatted soya bean powder have an extremely high tendency to foam and contain large amounts of water-insolubles, so uniform mixing is difficult to achieve in a large, outdoor, open reactor; CSL (corn steep liquor) which is the liquid by-product of sugar purification does not have uniform quality, especially in terms of the quantity of the microorganism that enters and this is likely to induce microbial contamination in the outdoor, open reactor for which it is difficult in to sterilize the medium. In addition, in order to minimize the entrance and growth of heterotrophic microorganisms which are the primary cause of microbial contamination and to ensure that the growth of those green algae which are capable of autotrophic growth by photosynthesis will predominate, it has been attempted to keep the medium ingredients in a state that is as oligotrophic as possible. For these reasons, only a few cases have been reported of using the organic nitrogen source in astaxanthin production by cultivation of green algae.
Patent Document 1: Japanese Patent No. 3163127
Patent Document 2: Japanese Patent Application 2002-294420
Non-Patent Document 1: Seibutsu Kogakukai-shi 71(4):233-237 (1993)
Non-Patent Document 2: Food Style 21, 5(12):25-35 (2001)
Non-Patent Document 3: NatuRose Technical Bulletin #78, Cyanotech Corporation (2000)
Non-Patent Document 4: F. Ferment. Technol. 74(1):17-20 (1992)
Non-Patent Document 5: Biotechnol. Lett. 19(6):507-509 (1997)
Non-Patent Document 6: Biotechnol. Lett. 16(2):133-138 (1994)
Non-Patent Document 7: J. Ferment. Bioeng. 74:17-20 (1992)
Non-Patent Document 8: J. Appl. Phycol. 5:593-604 (1993)
Non-Patent Document 9: J. Ferment. Bioeng. 82:113-118 (1996)

### DISCLOSURE OF THE INVENTION

### PROBLEMS TO BE SOLVED BY THE INVENTION

An object of the present invention is to provide a process which, by improving the conventional method of culturing the green alga *Haematococcus pluvialis,* promotes vegetative cell growth and astaxanthin biosynthesis so markedly as to enable efficient production of astaxanthin-containing lipids from the culture.

The present invention also provides a process that is free from the problem of microbial contamination accompanying the outdoor, open culture system and in which the green alga *Haematococcus pluvialis* is grown efficiently in an indoor reactor or in the outdoor, closed culture system so as to produce astaxanthin. As used herein, the term "closed system" means cultivation in a controlled environment within a closed or semi-closed vessel, which also means that the problem with the open system, or susceptibility to changes in the surrounding environment such as weather (e.g. air temperature, humidity, or the quantity of light) and microorganisms suspended in the air on account of the exposure of the green alga to an uncontrolled environment, is just a little or negligible.

### MEANS FOR SOLVING THE PROBLEMS

The present inventors noted the organic nitrogen source which had heretofore been considered to be unsuitable for use, and they conducted an intensive study on the effects it would have. As a result, they found that an organic nitrogen source having a low proportion of amino nitrogen to the total nitrogen (AN/TN ratio) was effective in the cell growth and astaxanthin production of the green alga *Haematococcus pluvialis.* The present invention has been accomplished on the basis of this finding.

The process of the present invention comprises cultivating the green alga *Haematococcus pluvialis* with an organic nitrogen source being used in a culture medium at an AN/TN ratio of 65% or less, preferably 43% or less, more preferably 35% or less, to obtain algal bodies in which astaxanthin-containing lipids have been accumulated. If necessary, the process may include the steps of extracting the astaxanthin-containing lipids from the algal bodies and optionally purifying the extracted lipids.

### Astaxanthin

Astaxanthin (3,3'-dihydroxy-β,β-carotene-4,4'-dione) that is biosynthesized by the green alga *Haematococcus pluvialis* is accumulated in its cells in two forms, free and ester. Ester forms include, for example, astaxanthin fatty acid monoester and astaxanthin fatty acid diester (J. Ferment. Bioeng. 71:335-339 (1991); Phytochemistry 20:2561-2564 (1981)). In the present invention, the astaxanthin-containing lipids mean those free forms of astaxanthin and/or ester forms of astaxanthin, which occur either singly or in admixture. The astaxanthin-containing lipids also mean mixtures of astaxanthin and fat-soluble components that are derived from algal bodies of *Haematococcus pluvialis* which contain astaxanthin.

### Green alga

The green alga *Haematococcus pluvialis* which is used in the present invention comprises a group of green algae that are unicellular which live in fresh water and may be exemplified by *Haematococcus pluvialis* ASTB BS2, CALU 9, CALU 333, CAUP G1002, CCAO, IBASU 38, IPPAS H-23, MUR 01, 02, 62, 63, 64, 65, 66, 67, 68, 69, 71, 72, 75, 76, 77, NIES 144, NIVA CHL9, and SMBA ("World Catalogue of Algae", p. 132-133, Japan Scientific Societies Press (1989)). Some strains of *Haematococcus lacustris* are the same as *Haematococcus pluvialis* and include ATCC 30402, SAG 34-1a, 1b, lc, 1d, 1e, 1f, 1h, 1k, 11, 1m, 1n, and UTEX 16 ("World Catalogue of Algae", p. 132-133, Japan Scientific Societies Press (1989)).

Vegetative cells of the green alga *Haematococcus pluvialis* usually occur as migratory cells each having two flagella, which grow by dividing within the wall of the parent cell as the green alga Chlamydomonas does. A vegetative cell is surrounded by a gelatin-like cell wall. Given appropriate culture conditions, the vegetative cell develops a thick, tough cell wall inside the gelatinous matter and the cell eventually stops migrating and forms a cyst (also known as aplanospore or akinete), either asexually or sexually, that has a hard cell wall and which can contain a large quantity of astaxanthin-containing lipids; given appropriate conditions, the cyst accumulates a significant amount of astaxanthin, changing from the brown-red immature cyst to the red mature cyst (J. Ferment. Bioeng. 84(1):94-97 (1997)). In this way, the life cycle of the green alga *Haematococcus pluvialis* involves vegetative cells and cysts but these can be easily differentiated by external observation.

### Medium

*Haematococcus pluvialis* may be cultured under the following conditions.

Basal medium: The basal medium may appropriately be selected from a variety of media employed in the growth of green algae, such as BM4 medium (for its composition, see Example 1), and the media described in Patent Documents 1 and 2, Non-Patent Documents 4-9, and Appl. Microbiol. Biotechnol., 53:530-535 (2000), as well as Appl. Microbiol. Biotechnol., 53:537-540 (2001).

Organic nitrogen source: According to the present invention, an organic nitrogen source having a low proportion of amino nitrogen to the total nitrogen (AN/TN ratio) is used as an organic nitrogen source to be added to the basal medium. The amino nitrogen as used herein refers to all forms of nitrogen other than that in the amino acid residues in peptides and it chiefly means the nitrogen in free amino acids. Typically, the AN/TN ratio of the organic nitrogen source is 65% or less, preferably 43% or less, and more preferably 35% or less.

The organic nitrogen source is preferably added to the medium at a concentration of 0.1 g/L or more.

The nitrogen source with an AN/TN ratio of 65% or less is exemplified by defatted soya bean powder, casein, and CSL (corn steep liquor), which may be used either individually or in combination of two or more members.
Soya bean peptides obtained by decomposing defatted soya bean powder, or peptone, triptone, polypeptone, etc. that are obtained by decomposing casein may also be used either individually or in combination of two or more members. Any organic nitrogen source that has an AN/TN ratio of 65% or less may be used without particular limitation; preferably, defatted soya bean powder or CSL is used either singly or in combination with other nitrogen sources. In addition to those nitrogen sources, conventionally known nitrogen sources (Japanese Patent No. 3163127) can also be used and they include inorganic nitrogen sources such as nitrates, urea and ammonium salts, amino acids like asparagine, glycine and glutamine, as well as organic nitrogen sources such as yeast extracts with AN/TN ratio in excess of 65%.

In order to confirm that a certain organic nitrogen source has an AN/TN ratio of 65% or less, preferably 43% or less, more preferably 35% or less, the amino nitrogen (AN) may be measured by the Van Slyke method and the total nitrogen (TN) by the Kjeldahl method and the proportions of the measured AN and TN values are calculated to determine the AN/TN of the particular organic nitrogen source.

Carbon source: The green alga *Haematococcus pluvialis* is a photosynthetic organism that is grown in the Nature with the aid of carbon dioxide and light energy. Therefore, it can be cultured under light conditions to grow autotrophically using carbon dioxide as a carbon source. In this case, microorganism growth can be effected without adding any carbon source to the medium; alternatively and if desired, cultivation may be performed under light conditions using the carbon sources noted below to effect heterotrophism. As used herein, the term "under light conditions" typically assumes the lightness in the day, but those areas of culture facilities which are brighter than the minimum lightness required for their inspection are included in the definition of "under light conditions".

The green alga *Haematococcus pluvialis* can also be cultured under dark conditions. In this case, it is necessary to use a medium in which a carbon source that can be substituted for carbon dioxide is contained in a sufficient amount to effect heterotrophism (Seibutsu Kogakukai-shi, 71(4):233-237 (1993)). Carbon sources that can be used include not only the conventionally known ones such as acetic acid, pyruvic acid, ethanol, and TCA-related organic acids (JP 11-56346 A) but also sugars, fatty acids, fatty acid esters, as well as oils and fats. Examples of TCA-related organic acids include citric acid, α-ketoglutaric acid, succinic acid, fumaric acid, and malic acid (Japanese Patent No. 3163127). Any one of these can be used as carbon sources but it is preferred to use acetic acid (J. Ferment. Bioeng. 74(1):17-20 (1992)).

The carbon sources are used in the medium in amounts of 0.01 g/L or more.

As will be understood by every skilled artisan, the term "cultivation under dark conditions" as used herein is intended as the collective name for covering not only complete darkness but also all cases where instead of applying intentional illumination, carbon sources are added to the medium to effect heterotrophic growth. Therefore, both temporary illumination and continuous, low-intensity illumination that are applied in checking the status of culture are also included in the definition of "cultivation under dark conditions" for the purposes of the present invention.

Aside from the medium components described above, inorganic salts that generate the ferric ion (Fe²⁺) or H₂O₂ may be added to give oxidative stress, thereby promoting astaxanthin synthesis (Seibutsu Kogakukai-shi, 71(4):233-237 (1993)).

### Pre-culture

In order to perform liquid culture of *Haematococcus pluvialis* and obtain its algal bodies or astaxanthin-containing lipids, stored algal bodies are first inoculated in a small volume of medium and then successively transferred to increasing volumes of medium to realize scale-up. Main culture means the final step of cultivation that is performed to recover the algal bodies or astaxanthin-containing lipids. Pre-culture, on the other hand, means cultivation by serial passage that is performed at each of the stages in the process of scaling up.

The conditions for pre-culture may be of any type; it may be under light conditions to effect autotrophic or heterotrophic growth; alternatively, it may be under dark conditions to effect heterotrophic growth. The cell morphology obtained by pre-culture may refer to vegetative cells or cysts. Since it is preferable to inoculate algal bodies as many as possible. It is preferred to perform pre-culture by employing vegetative cells of the greater proliferating ability.

For both pre-culture and the main culture that is described just below, the culture temperature is suitably at 15-25°C, preferably about 20°C, if vegetative cells are to be cultivated. Cysts are suitably cultivated at 25-35°C, preferably about 30°C.

### Main culture

A larger number of algal bodies are preferably transferred from the pre-culture to the main culture. Again, the morphology of the cells to be transferred from the pre-culture to the main culture may refer to vegetative cells or cysts, but encystment is preferably induced during the main culture so as to increase the astaxanthin content.

If the main culture is to be performed under dark conditions, aerobic conditions are required to induce encystment and increase the astaxanthin content. Means for creating aerobic conditions include, for example, diffusing oxygen into the culture solution from above, entrapping the air above the surface of the culture solution by shaking or agitating it, passing the air into the culture solution, and the combination of passing the air into the culture and agitating it; these methods may be applied either individually or in combination. In particular, aeration into the culture solution is preferred, with the air being preferably blown at relative rates of 0.01 vvm and more. Considering the aeration cost and the problem of foaming due to excessive aeration, the relative rate of aeration is preferably between 0.01 vvm and 1 vvm, more preferably between 0.1 vvm and 0.5 vvm.

When the main culture is performed under light conditions, encystment is induced spontaneously so as to increase the astaxanthin content.

### EFFECTS OF THE INVENTION

By performing cultivation with the novel medium composition, the process of the present invention obtains more algal bodies of *Haematococcus pluvialis* with a higher astaxanthin content during the main culture; as a result, cell growth and astaxanthin synthesis are increased to yield more astaxanthin-containing lipids. Compared to the conventional method of using inorganic nitrogen sources or using an organic nitrogen source with an AN/TN ratio in excess of 65%, the process of the present invention increases the production efficiency of astaxanthin-containing lipids by a factor of at least 1.5, say, between about 2 and 4. If the amount of astaxanthin produced in the present invention is expressed by the concentration per unit volume of the culture solution, it is at least 10 mg/L, preferably at least 15 mg/L, more preferably at least 20 mg/L, and most preferably at least 25 mg/L; if expressed by the amount per cell, the value is at least 40 pg/cell, preferably at least 50 pg/cell, more preferably at least 60 pg/cell, even more preferably at least 70 pg/cell, and most preferably at least 80 pg/cell. By thusly adjusting the AN/TN ratio of the culture medium, the production efficiency of astaxanthin-containing lipids or astaxanthin itself can be improved. This means the practical feasibility of using a small reactor. As a result, uniform mixing becomes possible and the product quality can be controlled to be constant.

### BEST MODE FOR CARRYING OUT THE INVENTION

A non-limiting specific example of the present invention that can attain its objects is described below with reference the case of cultivation under dark conditions. First, the green alga *Haematococcus pluvialis* is inoculated in a pre-culture medium. The pre-culture medium may be supplemented with 1.2 g/L of sodium acetate, 0.2 g/L of magnesium chloride hexahydrate, 0.01 g/L of ferrous sulfate heptahydrate, 0.02 g/L of calcium chloride dihydrate, 1 g/L of CSL, or 1.0 g/L of defatted soya bean powder. After inoculation in the medium, pre-culture (first stage) is started at a culture temperature of 20°C. In the pre-culture, the alga is grown as vegetative cells for four days. Then, the culture solution is transferred into a medium for the next (second) stage of pre-culture. The number of stages in the pre-culture may be determined as appropriate for the volume of the main culture solution and the same inoculation procedure is repeated for each of the stages in the pre-culture. For transfer from the pre-culture to the main culture, the pre-culture solution may be directly inoculated into the medium for main culture; preferably, however, the pre-culture solution is concentrated by centrifugation and decantation and the resulting suspension of algal bodies is inoculated into the medium for main culture. The medium for main culture may comprise an initial medium and an additional medium; the initial medium may be supplemented with 2.5 g/L of sodium acetate, 0.2 g/L of magnesium chloride hexahydrate, 0.01 g/L of ferrous sulfate heptahydrate, 0.02 g/L of calcium chloride dihydrate, 1 g/L of CSL, or 1.0 g/L of deffated soya bean powder; the additional medium may use sodium acetate at 3.7 g/L (as concentration per unit volume of the medium). After inoculating the medium for the main culture with algal bodies in that portion of the pre-culture solution which accounts for 10% of the main-culture medium, cultivation is performed for eight days at a culture temperature of 30°C with the air being blown at a relative rate of 0.5 vvm. Cultivation under dark conditions can be performed by essentially the same procedure as the above-described cultivation under light conditions, except that irradiation with light is carried out. The present invention is characterized in that cell growth and astaxanthin synthesis are promoted by using an organic nitrogen source with an AN/TN ratio of no more than 65% as a medium component, and by performing the culture method of the present invention, one can obtain astaxanthin-containing lipids in amounts that are much greater than are obtained in the case of using the conventional media.

### Recovery of algal bodies

The astaxanthin-containing lipids may be collected by the following procedure. First, after the end of the main culture, the culture solution, either as it is or after preliminary treatments such as sterilization and concentrating, is subjected to solid-liquid separation by a known suitable means such as sedimentation, centrifugation or filtration, so as to recover the algal bodies of *Haematococcus pluvialis.* To assist in solid-liquid separation, a flocculant or a filtering aid may be added. Exemplary flocculants include aluminum chloride, calcium chloride, sodium alginate, and chitosan. An exemplary filtering aid is diatomaceous earth. Then, the recovered algal bodies are preferably, but need not be, disrupted. The algal bodies can be disrupted by various known methods including grinding with added glass beads, application of osmotic pressure, using a French press, using a Manton-Gaulin homogenizer apparatus, freezing, using an extrusion granulator, and using a sonicator; these methods may be employed either singly or in combination. The algal bodies are preferably, but need not be, dried. The algal bodies may be dried by various known methods including fluidized-bed drying, spray drying, and freeze-drying; these methods may be employed either singly or in combination. If desired, the culture solution, either as it is or after preliminary treatments such as sterilization and concentrating, may be treated with a suitable apparatus such as a drum dryer or a granulator equipped with a heating capability; this enables the algal bodies to be recovered, disrupted and dried in one step.

The thus recovered algal bodies may be put to various uses as astaxanthin either directly (i.e. without being disrupted and dried) or after being properly treated (e.g., disrupted or dried). For example, they may be used as a pigment in feeds for fish in aquaculture.

### Extraction and purification

The algal bodies obtained by the above procedures may be subjected to extraction with an organic solvent (e.g. methanol, ethanol, hexane or acetone), or extraction with supercritical carbon dioxide, or extraction by pressing, whereupon the astaxanthin-containing lipids can be recovered. The obtained astaxanthin-containing lipids are mostly composed of astaxanthin fatty acid monoesters, with the other components being astaxanthin fatty acid diesters and the free form of astaxanthin (J. Ferment. Bioeng. 71:335-339 (1991); and Phytochemistry 20:2561-2564 (1981)).

In addition, the astaxanthin-containing lipids may be appropriately processed by known separating and purifying means such as liquid chromatography and molecular distillation, whereupon one can obtain astaxanthin per se or astaxanthin-containing lipids that have a desired purity of astaxanthin.

As described above, the developments of the photoreactor and the method of cultivation under dark conditions have begun to allow for the production of astaxanthin by indoor cultivation. In indoor cultivation and cultivation in an outdoor, closed system, a management of microorganisms such as medium sterilization can be performed more positive. As a result, organic nitrogen sources the use of which has been impossible in the conventional method of cultivation in an outdoor, open system on account of its constraints such as the potential microbial contamination can be employed according to the present invention under more desirably controlled conditions.

### EXAMPLES

### Haematococcus Culture Experiments

### Example 1: Effect of Medium Composition on Astaxanthin Production by Cysts

The green alga *Haematococcus pluvialis* NIES-144 was used. To prepare a medium for pre-culture, a basal medium (BM4 medium: 2.4 g/L of sodium acetate, 0.2 g/L of magnesium chloride hexahydrate, 0.001 g/L of ferrous sulfate heptahydrate, and 0.002 g/L of calcium chloride dihydrate) was supplemented with 2 g/L of yeast extract and 1 g/L of potassium nitrate, and after being adjusted to pH 6.8, the medium was sterilized at 121°C for 20 minutes. A portion (100 mL) of the prepared medium was placed in a 200-mL conical flask and subjected to stationary culture at 20°C for four days. For the subsequent main culture, 100 mL of each of the media shown in Table 1 below was placed in a 200-mL conical flask, inoculated with 10% (v/v) of the pre-culture solution, and subjected to shake culture at 30°C under dark conditions. At days 3 and 6 of the culture, sodium acetate was added at 3.7 g/L (as the concentration per unit volume of the culture solution) for 8-day culture.

The organic nitrogen sources used in the respective media had the following AN/TN ratios: 67% for the yeast extract; 33% for CSL; and 2% or less for the soya bean powder.

To measure the cell density, the culture solution was appropriately diluted to prepare a suspension which was loaded on a Thoma hemacytometer to count the cell number. To measure the concentration of astaxanthin, the cells were recovered by suction through a glass fiber filter, which was disrupted together with the cells in a mortar and subjected to extraction with acetone. By centrifugation, the supernatant of the acetone solution was obtained and its carotenoid concentration was determined by measuring the absorbance of the supernatant (at wavelengths of 480 nm and 750 nm) (see "A Practical Handbook of Sea Water Analysis", p. 185-206, Fisheries Research Board of Canada (1968)). Since most of the carotenoids accumulated in the cyst cells of *Haematococcus* had been verified to be astaxanthin (J. Ferment. Bioeng. 71(5):335-339 (1991)), the carotenoid concentrations obtained were expressed as astaxanthin concentrations.

After the culture, the algal bodies were recovered by centrifugation and subjected to acetone extraction so as to recover astaxanthin-containing lipids.

The astaxanthin concentrations obtained as the result of the culture are indicated below for the respective conditions: 1-1) 6.3 mg/L; 1-2) 10.8 mg/L; 1-3) 25.9 mg/L; 1-4) 21.5 mg/L; 1-5) 26.7 mg/L; and 1-6) 19.9 mg/L. For cell morphology, microscopic examination verified that cysts had formed under each condition.

No culture method of using CSL and soya bean powder to cultivate *Haematococcus* had conventionally been known. In Example 1, novel media supplemented with CSL and/or soya bean powder were employed to enable both the astaxanthin content per cell and the cell density to be increased; in addition, the increased astaxanthin concentrations allowed more astaxanthin-containing lipids to be recovered.

**Table 1**

| Condition No. | Medium composition | Astaxanthin concentration per unit volume of culture solution (mg/L) | Cell density per unit volume of culture solution (x 10⁵ cells/mL) | Astaxanthin content per cell content per cell (pg/cell) |
|---|---|---|---|---|
| 1-1) | BM4 medium + yeast extract 2 g/L + KNO₃ 1 g/L | 6.3 | 2.0 | 31.8 |
| 1-2) | BM4 medium + CSL 0.1 g/L | 10.8 | 2.6 | 41.5 |
| 1-3) | BM4 medium + CSL 1.0 g/L | 25.9 | 3.6 | 71.9 |
| 1-4) | BM4 medium + soya bean powder 0.1 g/L | 21.5 | 3.4 | 63.2 |
| 1-5) | BM4 medium + soya bean powder 1.0 g/L | 26.7 | 4.0 | 66.8 |
| 1-6) | BM4 medium + CSL 0.1 g/L + soya bean powder 0.1 g/L | 19.9 | 3.6 | 55.3 |

| | | | | |
|---|---|---|---|---|
| CSL: corn steep liquor Soya bean powder: defatted soya bean powder | | | | |

### Example 2: Effect of New Medium Composition on Vegetative Cell Growth

The green alga *Haematococcus pluvialis* NIES-144 was used.

Pre-culture was performed under comparable conditions to Example 1. For the subsequent main culture, 100 mL of each of the media shown in Table 2 below was placed in a 200-mL conical flask, inoculated with 10% (v/v) of the pre-culture solution, and subjected to stationary culture at 16°C under dark conditions for six days. The AN/TN ratios of the organic nitrogen sources were comparable to those found in Example 1.

The vegetative cell densities obtained by the cultures were 4.46 x 10⁵ cells/mL in the yeast extract/potassium nitrate medium, 7.07 x 10⁵ cells/mL in the CSL-supplemented medium, and 7.45 x 10⁵ cells/mL in the soya bean powder supplemented medium.

**Table 2**

| Condition No. | Medium composition | Cell density per unit volume of culture solution (x 10⁵ cells/mL) |
|---|---|---|
| 1-1) | BM4 medium + yeast extract 2 g/L + KNO₃ 1 g/L | 4.46 |
| 1-2) | BM4 medium + CSL 1.0 g/L | 7.07 |
| 1-3) | BM4 medium + soya bean powder 1.0 g/L | 7.45 |

| | | |
|---|---|---|
| CSL: corn steep liquor Soya bean powder: defatted soya bean powder | | |

### Example 3: Effect of New Medium Composition on Vegetative Cell Growth

The green alga *Haematococcus pluvialis* NIES-144 was used.

Pre-culture was performed under comparable conditions to Example 1. For the subsequent main culture, 100 mL of each of the media shown in Table 3 below was placed in a 200-mL conical flask, inoculated with 10% (v/v) of the pre-culture solution, and subjected to stationary culture at 20°C under dark conditions for five days.

The organic nitrogen sources used in the respective media had the following AN/TN ratios: 67% for the yeast extract; 40% for triptone; 38% for peptone; and 42.6% for polypeptone.

The vegetative cell densities obtained by the culture were 3.4 x 10⁵ cells/mL in the yeast extract/potassium nitrate medium, 5.7 x 10⁵ cells/mL in the triptone-supplemented medium, 7.4 x 10⁵ cells/mL in the peptone-supplemented medium, and 4.4 x 10⁵ cells/mL in the polypeptone-supplemented medium.

**Table 3**

| Condition No. | Medium composition | Cell density per unit volume of culture solution (x 10⁵ cells/mL) |
|---|---|---|
| 1-1) | BM4 medium + yeast extract 2 g/L + KNO₃ 1 g/L | 3.4 |
| 1-2) | BM4 medium + triptone 1.0 g/L | 5.7 |
| 1-3) | BM4 medium + peptone 1.0 g/L | 7.4 |
| 1-4) | BM4 medium + polypeptone 1.0 g/L | 4.4 |

### Example 4: Effect of Medium Composition on Astaxanthin Production by Cysts in Cultivation under Light Conditions

The green alga *Haematococcus pluvialis* NIES-144 was used.

To prepare a medium for pre-culture, a basal medium (BM4 medium: 2.4 g/L of sodium acetate, 0.2 g/L of magnesium chloride hexahydrate, 0.001 g/L of ferrous sulfate heptahydrate, and 0.002 g/L of calcium chloride dihydrate) was supplemented with 2 g/L of yeast extract and 1 g/L of potassium nitrate, and adjustment was made to pH 6.8. A portion (100 mL) of the prepared medium was placed in a 200-mL conical flask and subjected to stationary culture at 20°C for four days. For the subsequent main culture, 100 mL of each of the media shown in Table 4 below was placed in a 200-mL conical flask, inoculated with 10% (v/v) of the pre-culture solution, and subjected to shake culture at 30°C under light conditions (illuminance: 2000 lux). At day 3 of the culture, sodium acetate was added at 3.7 g/L (as the concentration per unit volume of the culture solution) for 6-day culture.

The astaxanthin concentrations obtained as the result of culture are indicated below for the respective conditions: 1-1) 8.2 mg/L; 1-2) 14.2 mg/L; 1-3) 32.0 mg/L; 1-4) 25.0 mg/L; 1-5) 32.0 mg/L; and 1-6) 24.1 mg/L. For cell morphology, microscopic examination verified that cysts had formed under each condition.

No culture method of using CSL and soya bean powder to cultivate *Haematococcus* had conventionally been known. In Example 4, novel media supplemented with CSL and/or soya bean powder were employed to enable both the astaxanthin content per cell and the cell density to be increased; in addition, the increased astaxanthin concentrations allowed more astaxanthin-containing lipids to be recovered.

**Table 4**

| Condition No. | Medium composition | Astaxanthin concentration per unit volume of culture solution (mg/L) | Cell density per unit volume of culture solution (x 10⁵ cells/mL) | Astaxanthin content per cell (pg/cell) |
|---|---|---|---|---|
| 1-1) | BM4 medium + yeast extract 2 g/L + KNO₃ 1 g/L | 8.2 | 2.2 | 37.3 |
| 1-2) | BM4 medium + CSL 0.1 g/L | 14.2 | 2.9 | 49.0 |
| 1-3) | BM4 medium + CSL 1.0 g/L | 32.0 | 3.9 | 82.1 |
| 1-4) | BM4 medium + soya bean powder 0.1 g/L | 25.0 | 3.6 | 69.4 |
| 1-5) | BM4 medium + soya bean powder 1.0 g/L | 34.1 | 4.5 | 75.8 |
| 1-6) | BM4 medium + CSL 0.1 g/L + soya bean powder 0.1 g/L | 25.5 | 3.9 | 65.4 |

| | | | | |
|---|---|---|---|---|
| CSL: corn steep liquor Soya bean powder: defatted soya bean powder | | | | |

### Example 5: Effect of New Medium Composition on Vegetative Cell Growth

The green alga *Haematococcus pluvialis* NIES-144 was used.

Pre-culture was performed under comparable conditions to Example 1. For the subsequent main culture, 100 mL of each of the media shown in Table 5 below was placed in a 200-mL conical flask, inoculated with 10% (v/v) of the pre-culture solution, and subjected to stationary culture at 16°C under light conditions (illuminance: 1500 lux) for three days.

The vegetative cell densities obtained by the culture were 4.3 x 10⁵ cells/mL in the yeast extract/potassium nitrate medium, 6.9 x 10⁵ cells/mL in the CSL-supplemented medium, and 7.3 x 10⁵ cells/mL in the soya bean powder supplemented medium.

**Table 5**

| Condition No. | Medium composition | Cell density per unit volume of culture solution (x 10⁵ cells/mL) |
|---|---|---|
| 1-1) | BM4 medium + yeast extract 2 g/L + KNO₃ 1 g/L | 4.3 |
| 1-2) | BM4 medium + CSL 1.0 g/L | 6.9 |
| 1-3) | BM4 medium + soya bean powder 1.0 g/L | 7.3 |

| | | |
|---|---|---|
| CSL: corn steep liquor Soya bean powder: defatted soya bean powder | | |

## Claims

1. A process for producing astaxanthin-containing lipids which comprises culturing green alga with an organic nitrogen source being used in a medium at an AN/TN ratio of 65% or less, preferably 43% or less, more preferably 35% or less, to obtain algal bodies in which astaxanthin-containing lipids have been stored.

2. The process according to claim 1, further including the steps of extracting the astaxanthin-containing lipids from the algal bodies and optionally purifying the extracted lipids.

3. The process according to claim 1, wherein the organic nitrogen source is at least one organic nitrogen source selected from the group consisting of corn steep liquor, soya bean powder, peptone, tripeptone, and polypeptone.

4. The process according to any one of claims 1 to 3, wherein the organic nitrogen source is used at least 0.1 g/L.

5. The process according to claim 4, wherein culture is performed in a reactor under the dark condition.

6. The process according to claim 5, wherein culture is performed under aerobic conditions.

7. The process according to claim 4, wherein culture is performed in a reactor under the light condition or in an outdoor, closed system.

8. The process according to claim 5 or 6, wherein astaxanthin is stored at a concentration of at least 10 mg/L of the culture solution or at least 40 pg/cell.
